Europäisches Patentamt

(19) European Patent Office     (11) Publication number: **0 228 732**
B1

Office européen des brevets

(12)      **EUROPEAN PATENT SPECIFICATION**

(45) Date of publication of the patent specification:     (51) Int. Cl.⁴: **C12P 19/14,** C12P 19/20,
28.02.90                                      C08B 30/04

(21) Application number: 86202156.5

(22) Date of filing: 03.12.86

(54) Improvements relating to the production of glucose syrups and purified starches from wheat and other cereal starches containing pentosans.

(30) Priority: 03.12.85 EP 85202017

(43) Date of publication of application:
15.07.87 Bulletin 87/29

(45) Publication of the grant of the patent:
28.02.90 Bulletin 90/9

(84) Designated Contracting States:
AT BE CH DE ES FR GB GR IT LI LU NL SE

(56) References cited:
FR-A- 2 555 602
US-A- 2 821 501

"Glucose syrups: science and technology", 1984,
pages 197,211-212,227-228, Elsevier Applied Science
Publishers, London, GB; D.J. FOLKES et al.: "Analysis
and characterisation of glucose syrups"
CARBOHYDRATE RESEARCH, vol. 118, 1983,
pages 215-231, Elsevier Science Publishers B.V.,
Amsterdam, NL; J. COMTAT: "Isolation, properties, and
postulated role of some of the xylanases from the
basidiomycete sporotrichum dimorphosporum"
CHEMICAL ABSTRACTS, vol. 97, no. 23, 6th
December 1982, page 458, abstract no. 196873g,
Columbus, Ohio, US; & JP-A-82 141 299 (AGENCY OF
INDUSTRIAL SCIENCES AND

(73) Proprietor: GIST-BROCADES S.A., Rue de Liège,
F-59121 Prouvy(FR)

(72) Inventor: Ducroo, Paul, Rue Mermoz 6,
F-59133 Phalempin(FR)

(74) Representative: Huygens, Arthur Victor, Dr. et al, c/o
Gist-Brocades N.V. Patent & Trademarks Department
Wateringseweg 1 PO Box 1, NL-2600 MA Delft(NL)

(56) References cited: (continuation)

TECHNOLOGY) 01-09-1982
CHEMICAL ABSTRACTS, vol. 97, no. 23, 6th
December 1982, page 458, abstract no. 196874b,
Columbus, Ohio, US; & JP-A-82 141 298 (AGENCY OF
INDUSTRIAL SCIENCES AND
TECHNOLOGY) 01-09-1982

## Description

The invention relates to the preparation of glucose syrups from unpurified wheat and other cereal starches containing pentosans and to the separation of such starches from other cereal constituents, for example gluten.

Cellulose and starch are the most abundant sources of carbohydrates. Because starch is much more readily acessible to the human digestive system than cellulose it has a long history as a food-stuff. It is also an important industrial raw material.

In principle starch can be depolymerized under the catalytic effect of acids but this route leads to a rather incomplete depolymerization and the formation of rather large amounts of by-products. Enzymatic hydrolysis of starch has therefore been receiving increasing attention.

Natural starch is known to contain two types of macromolecule composed of glucose units. One type of molecule, called amylose, is linear and consists exclusively of alpha,1-4 linked glucose units. Starch contains about 25% of amylose. The second type of molecule, amylopectin, is highly branched and contains alpha,1-4 as well as alpha,1-6 linked glucose units. The overall content of alpha,1-6 linkages is generally less than 5%.

In modern industrial starch degradation two types of enzyme are in common use. The enzyme alpha-amylase is used for the liquefaction (or thinning) of starch into dextrins having an average degree of polymerization of about 7-10, and the enzyme amyloglucosidase is used for the final saccharification, resulting in a syrup of high glucose content (92-96%).

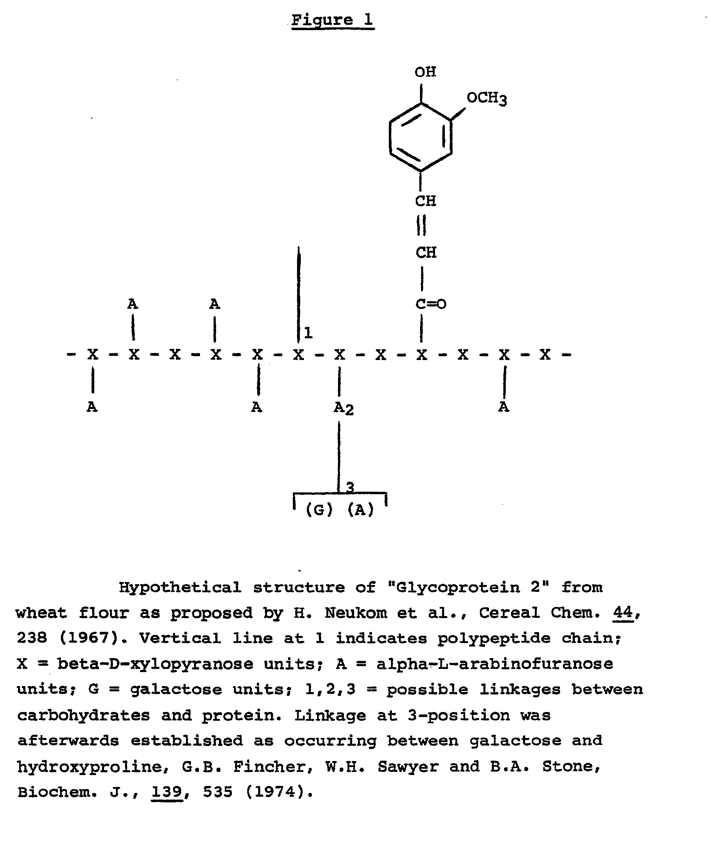

Cereals such as wheat and barley contain gums which increase the viscosity and reduce the filterability of aqueous extracts of the cereals including glucose syrups made in the manner just described and purified starches themselves. Such gums consist mainly of glucans, but also contain some pentosans. The structure of pentosans is more complicated than the usually presented structure with long chains of 1,4-beta-D-xylopyranose and single 1,2- or 1,3-alpha-L-arabinofuranose side groups (in the ratio of 1 arabinose to 2 xylose units); see Figure 1 of the accompanying drawings, taken from H. Neukom, L. Providoli, H. Gremli and P.A. Jui, Cereal Chem., 44, 238 (1967).

The properties of pentosans vary with the presence or absence of peptides, ferulic acid and arabinogalactan. About 2/3 of total pentosans are insoluble because of their high molecular weight and some interlinkages with proteins and other constituents. They have a very high water retention power and give very bulky spent filtration cakes. When arabinofuranose side groups of soluble pentosans are hydrolysed, an association and precipitation of non-substituted xylans is observed.

The averave pentosan content of various cereals is as follows (see "Handbuch der Lebensmittelchemie", Vol. 5, p. 32, 1967, Springer Verlag):

| Cereal grain | Pentosans (% dry weight) |
|---|---|
| Barley (incl. husks) | 10.3 |
| Wheat | 7.4 |
| Rye | 10.6 |
| Oats (incl. husks) | 7.5 |
| Corn | 6.2 |
| Rice | 2.0 |
| Millet | 2.0 |

In order to improve the filterability of glucose syrups obtained from unpurified wheat starch, treatment of the syrup with xylanase has been tried. Xylanase is an enzyme which hydrolyses xylans which occur in pentosans.

In Starch 36, 135 (1984), a beta-glucanase product of fungal origin is described which possesses pentosanase activity. This enzyme is recommended for the treatment of waste water from the wheat starch industry.

British Patent Specification No. 2,150,933 describes a pentosanase obtained by fermentation of Talaromyces (i.e. Penicillium) emersonii. This enzyme is stated to be capable of catalysing the degradation of xylan and to be useful, interalia, for reducing the viscosity of starch slurries to improve starch recovery.

According to the present invention, glucose syrups of improved filterability and/or lower viscosity are produced from impure cereal starch containing pentosans by a process which comprises subjecting the said impure starch to the action of Disporotrichum xylanase to hydrolyse pentosans and to a hydrolysis to convert starch into glucose.

2

Thus the preparation of a glucose syrup from unpurified wheat starch may be improved by hydrolysing the starch with an enzyme mixture which contains, in addition to alpha-amylase and/or amyloglucosidase, also Disporotrichum xylanase.

The xylanase can also be used in the recovery of starch from wheat or other flours obtained from cereals containing pentosans. In the wet milling of wheat, gluten is separated from other constituents (starch slurry) mechanically. The starch slurry separates into a tightly packed lower layer of purified starch and a supernatant mucilaginous material. This mucilaginous fraction which contains small starch granules, hemicellulose and protein, has been called squeegee, tailings or sludge. The hemicellulose portion contains xylose (about 60%), arabinose (about 38%) and glucose (about 2%).

By passing wheat starch slurry through a continuous centrifuge, producers are able to separate the purer concentrate or "A" starch stream while the small grains together with swollen damaged grains and pentosan complexes form the impure or "B" starch stream. After previous treatment of the wheat starch slurry with Disporotrichum xylanase before passage through the continuous centrifuge, the yield of "A" quality starch is increased by hydrolysis of the pentosans and reduction of the viscosity of the tailings.

The xylanase used in the present invention is an endo-xylanase obtained from the Basidiomycete Disporotrichum and specifically Disporotrichum dimorphosporum, described in a revision of the genus Sporotrichum by J.A. Stalpers, Studies in Mycology, 24, 1 (1984).

Preferably, a xylanase preparation is used derived from Disporotrichum dimorphosporum. Very satisfactory results are obtained when using a xylanase preparation derived from Disporotrichum dimorphosporum strain ATCC 24562, available from the American Type Culture Collection, which is identical with strain CBS 484.76, available from the Centraal Bureau voor Schimmelcultures, Baarn, Netherlands. These strains are preferred for the purpose of this invention.

Other xylanase preparations which may be used according to the present invention are those having substantially the same characteristics as the xylanase preparation which is obtainable from Disporotrichum dimorphosporum strain ATCC 24562 (or CBS 484.76). This includes preparations obtained from a transformed host microorganism containing the gene coding for the xylanase produced by said Disporotrichum dimorphosphorum strain ATCC 24562 (or CBS 484.76).

By culturing on a medium containing cellulose, pectin, yeast extract and different salts, Disporotrichum dimorphosporum produces an endo-xylanase. Endo-xylanase hydrolyses the beta, 1—4-xylose bindings within the pentosan chains. From a technical point of view, an endo-type enzyme is generally preferable because it hydrolyses high molecular weight polysaccharides very rapidly. An exo-type enzyme requires more time and more enzymatic concentration in order to reach the same technological result.

In our co-pending patent application, EP-A 0 227 159 a method is disclosed for determining endo-xylanase activity. This application is herein incorporated by reference.

A concentrate of Disporotrichum xylanase suitable for use in the present invention may be obtained in the following manner. The fermentation is carried out in a sterile tank and medium in known manner. The culture medium contains cellulose, pectin, yeast extract and appropriate salts. It is inoculated with a pure culture of Disporotrichum dimorphosporum. The fermentation is effected at a constant temperature between 20°C and 37°C, preferably about 32°C, and the pH is maintained within the range of 3.0 to 6.0, preferably 4.0 to 4.5. The fermentation can be batchwise or continuous. The xylanase activity is followed during the process. It is not necessary to induce the production of the enzyme by addition of xylan-containing materials (e.g. corn cobs or flours), and the addition of such products mainly promotes the formation of an exoxylanase, which is less useful for the invention. When the required enzymatic activity has been reached the mash is harvested, filtered and concentrated by vacuum concentration or ultrafiltration. The concentrate can be sold as a liquid preparation or spray dried in a powder form. The endoxylanase hydrolyses the beta 1—4-xylose linkages within the pentosan chains.

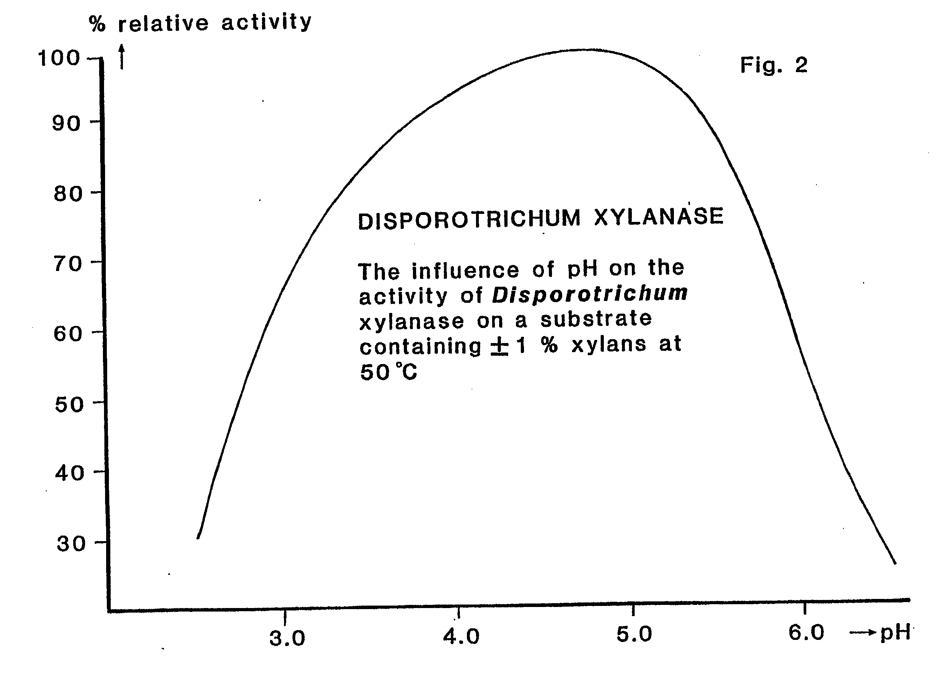

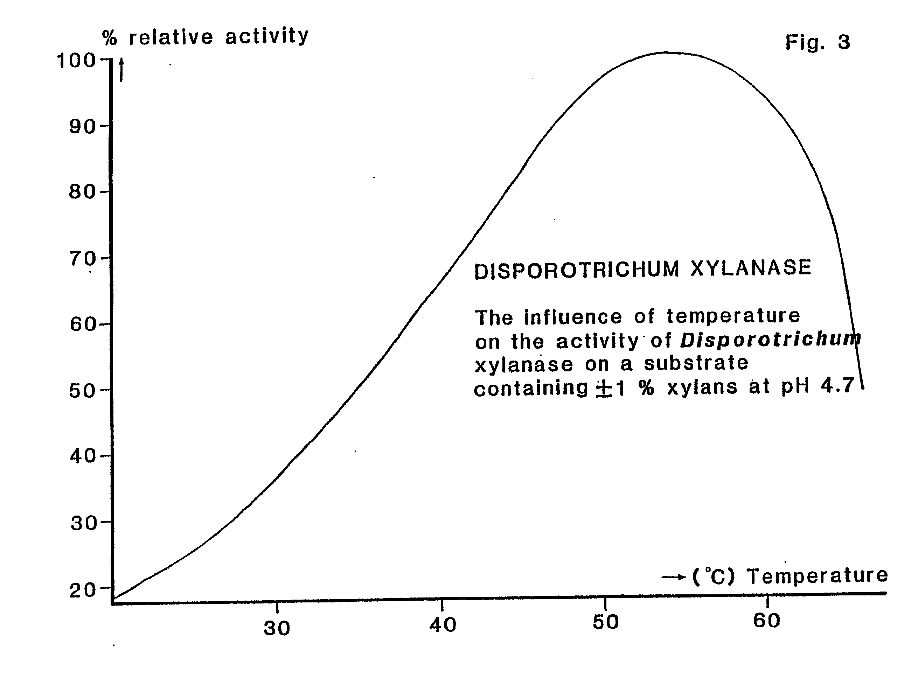

The properties of the non-purified enzymatic product have been studied viscosimetrically with a substrate containing ± 1% xylans (see accompanying Figures 2 ad 3). The optimum pH is 4.7, but between pH 3.0 and 6.0 the relative activity is more than 50%. The optimum temperature is 55°C but more than 50% relative activity remains at 65°C. Purification of this Disporotrichum xylanase was studied by Comtat et al. (J. Comtat, K. Ruel, J.-P. Joseleau and F. Barnoud, Symposium on Enzymatic Hydrolysis of Cellulose, S.I.T.R.A., Helsinki, Finland, 351 (1975); J. Comtat and J.-P. Joseleau, Carbohydr. Res., 95, 101 (1981)).

When the influence of enzymes with endo- and exo-xylanase activity on the filterability of glucose syrups made from unpurified wheat starch was investigated, it was found that Sumizym AC (Aspergillus niger cellulase from Shin Nihon), Drum pectinase (Aspergillus niger drum pectinase from Gist- brocades) and Disporotrichum xylanase were very active. Results obtained with Sumizym AC are highly dependent on enzymatic concentration, but the results obtained with Disporotrichum xylanase are not. It appears that Sumizym AC is only effective by its exo-enzymatic activity and Disporotrichum xylanase only by its endo-enzymatic activity.

The total effect of Disporotrichum xylanase is apparently obtained at low concentration. Drum pectinase and Sumizym AC have about the same efficiency but Drum pectinase gives more glucose reversion.

Disporotrichum endo-xylanase and Penicilliumemersonii endo-xylanase were compared by addition of the same quantities of exo-xylanase activity from Aspergillus niger products. P. emersonii xylanase and exo-xylanase blends show a smaller effect than Disporotrichum xylanase and exo-xylanase blends.

The following Examples illustrate the invention.

Example I

Standard procedure for liquefaction, saccharification and filtration of type B wheat starch

As substrate, type B wheat starch from Roquette Frères, reference LAB 833, containing about 1.2-1.5% proteins and 3-4% pentosans, was used.

A. Liquefaction process.

Wheat starch was liquefied at 30% D.S. (dry sdids) in well water (160 ppm CA++) in a liquefaction pilot plant according to the following process:
6 minutes at 105 -106°C
2 hours at 95°C

We have used 6 units of Maxamyl, a thermostable Bacillus licheniformis alpha-amylase from Gist-brocades, per gram of D.S. The pH was corrected to 6.4 with NaOH. It was not possible to liquefy at higher D.S. because the slurry was very viscous.

B. Saccharification process.

The pH of liquefied starch was corrected to 4.2. For the first experiments, 25000 AGI of Amigase GM (an Aspergillusniger amyloglucosidase from Gist-brocades) per kg D.S. was used. This extra dosage was chosen to avoid interference of starch retrogradation on the filtration tests. The last experiments were realized with the normal 17500 AGI/kg D.S.

Filtration enzymes have been added with the Amigase at the beginning of saccharification.

After 3 days at 60°C, the filterability was tested.

AGI (one unit of amylo-glucosidase activity) as defined in European Patent No. 0 140 410 is the amount of enzyme that releases 1 $\mu$ md of dextrose from soluble starch (100% of dry matter) per minute at 60°C under optimum conditions of starch degradation.

C. Filtration tests and analysis.

A Seitz laboratory filter maintained at 60°C by water circulation, equipped with a cloth filter and Dicalite 4258 S2 (Kieselguhr), was used. A precoat was made with 15 g of Dicalite 4258 S2 dispersed into 150 ml glucose syrup at 30% D.S. The saccharified starch was filtered under 1 bar pressure and the volume of filtrate per unit time was measured. A coefficient of filterability was obtained from the formula:

$$\frac{\text{volume of filtrate after 15min} - \text{volume of filtrate after 1min}}{14}$$

= volume of filtrate per min.

After filtration, the viscosity was measured in a capillary viscosimeter at 20°C and HPLC analysis was used for determination of sugars.

Example II

Improvement of the filtration of liquefied wheat starch by the addition of Disporotrichum xylanase.

Type B wheat starch containing 2-3% pentosans was liquefied by the traditional process used for corn starch syrup with 1° liquefaction of solid starch with alpha-amylase and 2° saccharification of the resulting liquefied starch with amyloglucosidase. This gave a syrup of high glucose content. Disporotrichum xylanase was introduced at the saccharification stage in addition to the amyloglucosidase.

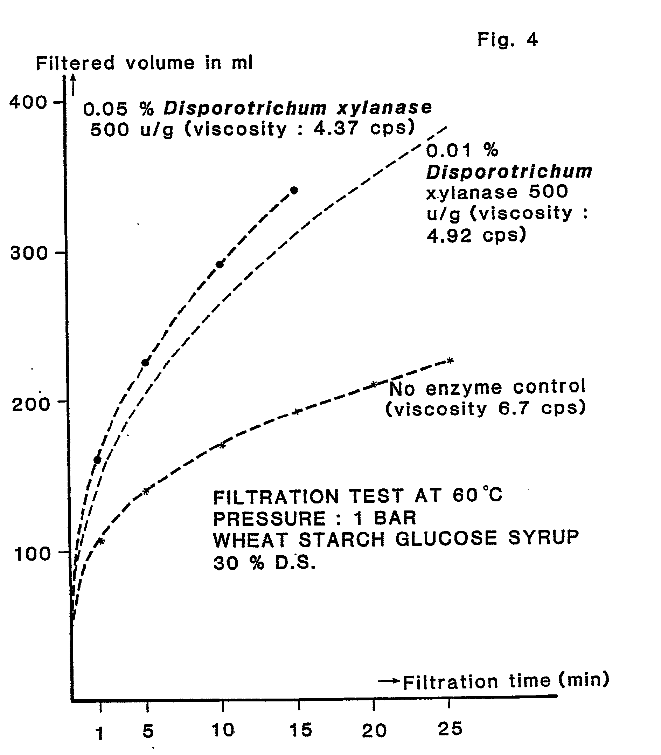

After three days of incubation at 60°C and a pH of 4.2-4.5, filtration tests were carried out under standard conditions. The results, which are given in Figure 4, show that the addition of Disporotrichum xylanase decreases the syrup viscosity, and thus improves the filterability.

## Example III

### The effect of Disporotrichum xylanase and Sumizym AC on the filtration of liquefied type B wheat starch

Conditions as in Example I, with 25000 AGI/kg D.S.

| enzyme | dosage in % D.S. | volume of filtrate per minute |
|---|---|---|
| Blank (only AG) | – | 7.8 ml |
| Disporotrichum xylanase | | |
| diluted to 500 u/g | 0.05 | 15.6 ml |
| diluted to 500 u/g | 0.1 | 15.2 ml |
| diluted to 500 u/g | 0.2 | 16.3 ml |
| Sumizym AC | 0.005 | 15.3 ml |
| Sumizym AC | 0.01 | 18.3 ml |
| Sumizym AC | 0.02 | 21.8 ml |
| Sumizym AC | 0.05 | 24.5 ml |

Results obtained with Sumizym AC are highly dependent on enzymatic concentration, but the results obtained with Disporotrichum xylanase are not.

## Example IV

### The effect of Disporotrichum xylanase + Sumizym AC or Drum pectinase blends on the filtration of liquefied type B wheat starch

Conditions as in Example I, with 25000 AGI/kg D.S.

| first enzyme | dosage in % D.S. | second enzyme | dosage in % D.S. | volume of filtrate per minute |
|---|---|---|---|---|
| blank | – | – | – | 7.5 ml |
| Disporotrichum xylanase | | | | |
| diluted to 500 u/g | 0.005 | – | – | 12.9 ml |
| | 0.01 | – | – | 14.0 ml |
| | 0.02 | – | – | 14.8 ml |
| | 0.05 | – | – | 15.5 ml |
| | 0.05 | Sumizym AC | 0.005 | 18.5 ml |
| | 0.05 | Sumizym AC | 0.01 | 21.0 ml |
| | 0.05 | Sumizym AC | 0.02 | 20.8 ml |
| | 0.05 | Drum pect. | 0.005 | 17.8 ml |
| | 0.05 | Drum pect. | 0.01 | 20.5 ml |
| | 0.05 | Drum pect. | 0.02 | 21.4 ml |

The total efficiency of Disporotrichum xylanase is obtained at low concentration. When used with this endo-xylanase, exo-xylanases from Drum pectinase and Sumizym AC have about the same efficiency but Drum pectinase gives more glucose reversion.

Example V

The effect of Disporotrichum xylanase + Sumizym AC or Drum pectinase blends on the filtration of liquefied type B wheat starch and on the glucose yied

Conditions as in Example I with 25000 AGI/kg D.S.

| first enzyme | dosage in % D.S. | second enzyme | dosage in % D.S. | volume of filtrate per min (ml) | viscosity (mPa.s) | glucose yield (%) |
|---|---|---|---|---|---|---|
| blank (with AG) | – | – | – | 6.40 | 6.8 | – |
| Disporotrichum xylanase | | | | | | |
| diluted to 500 u/g | 0.01 | – | – | 11.20 | 4.45 | – |
| diluted to 500 u/g | 0.01 | Drum p. | 0.005 | 14.80 | 3.71 | 91.49 |
| diluted to 500 u/g | 0.01 | Drum p. | 0.0075 | 15.20 | 3.72 | 90.67 |
| diluted to 500 u/g | 0.01 | Drum p. | 0.01 | 16.4 | 3.71 | 90.40 |
| diluted to 500 u/g | 0.01 | Drum p. | 0.0125 | 16.8 | 3.69 | 90.02 |
| diluted to 500 u/g | 0.01 | Sumizym AC | 0.01 | 17.5 | 3.59 | 91.96 |

This experiment confirms the high reversion effect on glucose yield of Drum pectinase. The test with
Sumizym AC was carried out for comparison.

Example VI

The effects of Disporotrichum xylanase and Penicillium emersonii beta-glucanase with the same addition
of exo-xylanase activity

Conditions as in Example I, with 17500 AGI/kg D.S.

| first enzyme | dosage in % D.S. | second enzyme | dosage in % D.S. | volume of filtrate per min (ml) | viscosity (mPa.s) | glucose yield (%) |
|---|---|---|---|---|---|---|
| blank (with AG) | – | – | – | 3.5 | 6.83 | 93.13 |
| Disporotrichum xylanase | | | | | | |
| diluted to 500 u/g | 0.01 | – | – | 9.9 | 4.40 | 92.17 |
| diluted to 500 u/g | 0.01 | Drum p. | 0.005 | 15.2 | 4.22 | 90.97 |
| diluted to 500 u/g | 0.01 | Sumizym AC | 0.0035 | 15.4 | 4.12 | 91.58 |
| P. emersonii | | | | | | |
| beta-glucanase | 0.02 | – | – | 9.0 | 4.67 | 92.28 |
| beta-glucanase | 0.02 | Drum p. | 0.005 | 11.7 | 4.35 | 90.96 |
| beta-glucanase | 0.02 | Sumizym AC | 0.0035 | 11.8 | 4.22 | 91.74 |

These experiments show a lower synergic effect for P. emersonii beta-glucanase endo-xylanase +
exo-xylanase blends in comparison with Disporotrichum xylanase + exo-xylanase blends.

**Claims**

1. Process for producing a glucose syrup of improved filterability and/or lower viscosity from impure
cereal starch containing pentosans which comprises subjecting the said impure starch to the action of
Disporotrichum xylanase to hydrolyse pentosans and to a hydrolysis to convert starch into glucose.

2. Process according to Claim 1 in which glucose syrup is produced by subjecting impure wheat starch
to the action of alpha-amylase and/or amyloglucosidase, and the said xylanase.

3. Process according to claim 1 or 2 in which the said xylanase is derived from Disporotrichum dimorphosporum ATCC 24562.

4. Process according to any one of claims 1 to 3 in which the said xylanase has substantially the same
characteristics as the xylanase which is obtainable from Disporotrichum dimorphosporum ATCC 24562.

5. Process according to any one of claims 1 to 4 in which the xylanase is used in admixture with Aspergillus niger exo-xylanase.

6. Process for separating a cereal starch from other constituents of the cereal which comprises subjecting a crude slurry of cereal starch to the action of <u>Disporotrichum</u> xylanase before the starch is separated mechanically from the other constituents of the slurry.

**Revendications**

1. Procédé pour produire un sirop de glucose ayant une aptitude améliorée à la filtration et/ou une plus faible viscosité à partir d'amidon céréalier impur contenant des pentosanes, qui comprend l'exposition de cet amidon impur à l'action de la xylanase de <u>Disporotrichum</u> pour hydrolyser les pentosanes et à une hydrolyse pour convertir l'amidon en glucose.

2. Procédé suivant la revendication 1, dans lequel le sirop de glucose est produit en exposant de l'amidon de blé impur à l'action de l'alpha-amylase et/ou de l'amyloglucosidase, et de cette xylanase.

3. Procédé suivant la revendication 1 ou 2, dans lequel la xylanase dérive de <u>Disporotrichum dimorphosporum</u> ATCC 24562.

4. Procédé suivant l'une quelconque des revendications 1 à 3, dans lequel la xylanase a sensiblement les mêmes caractéristiques que la xylanase qui peut être obtenue à partir de <u>Disporotrichum dimorphosporum</u> ATCC 24562.

5. Procédé suivant l'une quelconque des revendications 1 à 4, dans lequel la xylanase est utilisée en mélange avec de l'exo-xylanase d'<u>Aspergillus niger</u>.

6. Procédé pour séparer un amidon céréalier d'autres constituants de la céréale, qui comprend l'exposition d'une dispersion brute d'amidon céréalier à l'action de la xylanase de <u>Disporotrichum</u> avant de séparer l'amidon mécaniquement des autres constituants de la dispersion.

**Patentansprüche**

1. Verfahren zur Herstellung eines Glucosesirups mit verbesserter Filtrierbarkeit und/oder niedriger Viskosität aus unreiner Getreidestärke, die Pentosane enthält, dadurch gekennzeichnet, daß man die unreine Stärke der Einwirkung von Disporotrichum-Xylanase unterwirft, um Pentosane zu hydrolysieren, und einer Hydrolyse unterwirft, um Stärke in Glucose überzuführen.

2. Verfahren nach Anspruch 1, dadurch gekennzeichnet, daß Glucosesirup hergestellt wird, indem man unreine Weizenstärke der Einwirkung von alpha-Amylase und/oder Amyloglucosidase und der genannten Xylanase unterwirft.

3. Verfahren nach Anspruch 1 oder 2, dadurch gekennzeichnet, daß die genannte Xylanase aus Disporotrichum dimorphosporum ATCC 24562 stammt.

4. Verfahren nach Anspruch 1 oder 2, dadurch gekennzeichnet, daß die genannte Xylanase im wesentlichen die gleichen Eigenschaften hat wie die Xylanase, die aus Disporotrichum dimorphosporum ATCC 24562 erhältlich ist.

5. Verfahren nach einem der Ansprüche 1 bis 4, dadurch gekennzeichnet, daß die Xylanase im Gemisch mit Aspergillus niger-Exo-Xylanase verwendet wird.

6. Verfahren zur Abtrennung einer Getreidestärke von anderen Bestandteilen des Getreides, dadurch gekennzeichnet, daß man eine rohe Getreidestärkemilch der Einwirkung von Disporotrichum-Xylanase unterwirft, ehe die Stärke mechanisch von den anderen Bestandteilen der Stärkemilch abgetrennt wird.

## Figure 1

```
                                        OH
                                        |
                                        /\  OCH3
                                       |  |
                                        \/
                                        |
                                        CH
                                        ||
                                        CH
                                        |
              A         A               C=O
              |         |       |1      |
 - X - X - X - X - X - X - X - X - X - X - X - X -
     |             |       |               |
     A             A       A2              A
                           |
                           |
                           |3
                         __|__
                        | (G) (A) |
```

Hypothetical structure of "Glycoprotein 2" from wheat flour as proposed by H. Neukom et al., Cereal Chem. **44**, 238 (1967). Vertical line at 1 indicates polypeptide chain; X = beta-D-xylopyranose units; A = alpha-L-arabinofuranose units; G = galactose units; 1,2,3 = possible linkages between carbohydrates and protein. Linkage at 3-position was afterwards established as occurring between galactose and hydroxyproline, G.B. Fincher, W.H. Sawyer and B.A. Stone, Biochem. J., **139**, 535 (1974).

% relative activity

**DISPOROTRICHUM XYLANASE**

The influence of pH on the activity of *Disporotrichum* xylanase on a substrate containing ± 1 % xylans at 50 °C

Fig. 2

EP 0 228 732 B1

**% relative activity**

**DISPOROTRICHUM XYLANASE**

The influence of temperature
on the activity of *Disporotrichum*
xylanase on a substrate
containing ±1 % xylans at pH 4.7

→ (°C) Temperature

Fig. 3

EP 0 228 732 B1

Fig. 4

Filtered volume in ml

0.05 % *Disporotrichum xylanase* 500 u/g (viscosity : 4.37 cps)

0.01 % *Disporotrichum* xylanase 500 u/g (viscosity : 4.92 cps)

No enzyme control (viscosity 6.7 cps)

FILTRATION TEST AT 60 °C
PRESSURE : 1 BAR
WHEAT STARCH GLUCOSE SYRUP
30 % D.S.

→ Filtration time (min)